# EUROPEAN PATENT APPLICATION

(11) **EP 1 621 191 A1**
(43) Date of publication of application: **01.02.2006**
(21) Application number: 04017927.7
(22) Date of filing: 29.07.2004
(51) Int. Cl.: A61K 31/19, A61P 3/00, A61P 11/00, A61P 13/00, A61P 17/00, A61P 19/00, A61P 35/00

(54) **Treatment of inflammatory diseases by RXR Antagonists**

(71) Applicant: Bollag, Werner, 4054 Basel (CH)
(72) Inventor: Bollag, Werner, 4054 Basel (CH)
(74) Representative: Maucher, Wolfgang

(57) **Abstract**

Retinoids with retinoid antagonistic activities, especially Retinoid X Receptor antagonists called RXR antagonists, pharmaceutically acceptable salts and pharmaceutically acceptable esters and amides thereof, have been found to be efficacious in the treatment of inflamatory diseases of the skin and mucous membranes, and of other tissues and organs especially by topical or oral administration of RXR antagonists.

## Description

### Summary of the invention

The present invention relates to the use of retinoid antagonists comprising retinoids with selective Retinoic Acid Receptor (RAR) antagonistic activity, Retinoid X Receptor (RXR) antagonistic activity or mixed RAR-RXR antagonistic activity, for the manufacture of a medicament for the treatment of one or more (= an) inflammatory diseases of the skin and/or mucous membranes and of other tissues and organs, as well as to the use of such retinoid antagonists for the treatment of any one or more of these diseases, to a method of treatment for said diseases comprising administering such a retinoid antagonist to a patient, to the use in the treatment of such a retinoid antagonist for the treatment of said diseases, to such a retinoid antagonist for use in the treatment of one or more of said diseases and/or to a pharmaceutical composition for use in the treatment of any one or more of said diseases comprising such a retinoid antagonist.

### Background of the invention

Retinoids are a class of compounds structurally related to vitamin A, comprising natural and synthetic compounds. A series of retinoids have been found to be clinically useful in the treatment of dermatological and oncological diseases.

The activity of retinoids is thought to be mediated by the nuclear retinoid receptors RAR α, β, γ and/or RXR α, β, γ belonging to the superfamily of steroid, thyroid hormone, vitamin D, peroxisome proliferator-activated receptors (Pfahl et al., Vitamins and Hormones 49, 327-382 (1994). Retinoids with receptor agonistic activity bind and activate receptors, whereas retinoids with receptor antagonistic activity bind receptors but do not activate them.

Experimentally, retinoids with retinoid receptor antagonistic activity (retinoid antagonists) are effective in counteracting many properties of retinoids with retinoid receptor agonistic activity (retinoid agonists) such as inhibition of cell proliferation, induction of cell differentiation, induction of apoptosis and inhibition of angiogenesis (Bollag et al., Int.J.Cancer 70, 470-472 (1997). Retinoid antagonists are also suppressing toxic side effects of retinoid agonists such as the signs and symptoms of the hypervitaminosis A syndrome and terato-genesis (Standeven et al., Toxicol. Appl. Pharmacol. 138, 169-175 (1996); Eckhardt and Schmitt, Toxicol. Letters 70, 299-308 (1994). Therefore, they may be useful clinically in preventing or treating adverse events caused by retinoid agonists.

Retinoid antagonists have been proposed for clinical use in prevention and therapy of retinoid-induced toxicity and side effects, particularly of the so-called hypervitaminosis A syndrome. Retinoid antagonists have also been proposed to be used in combination with retinoid receptor agonists or other nuclear receptor agonists for prevention and treatment of preneoplastic or neoplastic lesions, vitreo-retinopathy and retinal detachement. In addition, retinoid antagonists could be used as single agents, based on their anti-proliferative effect, for treatment of certain neoplasms insensitive to retinoid receptor agonists (see WO 97/09297).

Furthermore, retinoid antagonists have been found to be efficacious in experimental models predictive for the treatment of T-helper cell type 2 (Th2)-mediated immune diseases, or immunoglobulin E (IgE)-mediated diseases, allergic diseases, atopic diseases or diseases mediated by the Th2-related cytokines. They encompass atopic dermatitis (neuroder-mitis), allergic rhinitis or hay fever and allergic bronchial asthma (see WO 99/24024 and WO 00/53562).

### General description of the invention

For the first time, quite unexpectedly, it has now been found that retinoid antagonists, in particular RXR antagonists, are useful in the treatment of inflammatory diseases of the skin and/or mucous membranes, and especially of other tissues and organs, by all kinds of pharmaceutical administration, but in particular by topical application to the skin and mucous membranes.

### Detailed description of the invention

In the subsequent detailed specification, whereever the term USE is employed, this refers to the use of retinoid antagonists comprising retinoids with selective Retinoic Acid Receptor (RAR) antagonistic activity, Retinoid X Receptor (RXR) antagonistic activity or mixed RAR-RXR antagonistic activity, for the manufacture of a medicament for the treatment of one or more inflammatory diseases of the skin and/or a (= one or more) mucous membrane and of other tissues and organs, especially those mentioned as preferred below, the use of such retinoid antagonists for the treatment of any one or more of these diseases, to a method of treatment for said diseases comprising administering such a retinoid antagonist to a patient especially to a patient in need of such treatment in a dose that is effective in said treatment, to the use in the treatment of such a retinoid antagonist for the treatment of said diseases, to such a retinoid antagonist for use in the treatment of one or more of said diseases and/or to a pharmaceutical composition for use in the treatment of any one or more of said diseases comprising such a retinoid antagonist preferably in an amount effective in said treatment, if not indicated otherwise.

In the scope and disclosure of the present invention, the term "retinoid antagonists" is used for retinoids or compounds with RAR, preferably RXR or mixed RAR-RXR antagonistic activity.

Besides the other RAR antagonists described in WO 99/24024 and WO 00/53562, which are herewith incorporated by reference with regard to these other compounds and the compound classes mentioned therein, the present invention relates in particular to the USE any one or more of the following compounds:
A compound of the formula I, 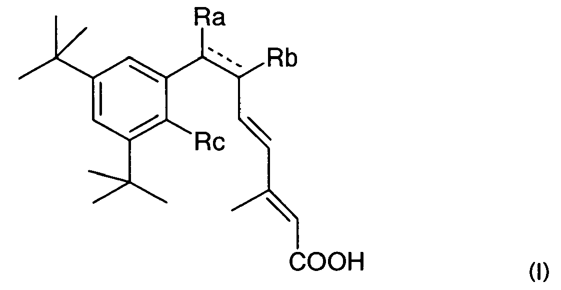 wherein the dotted line represents a bond (thus together with the solid line forming a double bond between the carbon atoms carrying Ra and Rb) or is absent (thus forming a single bond), and when the dotted bond is present, Ra is methyl and Rb is hydrogen, when the dotted bond is absent, Ra and Ra together are methylene thus forming, with the two carbon atoms carrying Ra and Rb, a preferably cis-substituted cyclopropyl ring; and Rc is C₁-C₄-alkoxy; the synthesis of these compounds is disclosed in US 6,326,397;
a compound of the formula II, 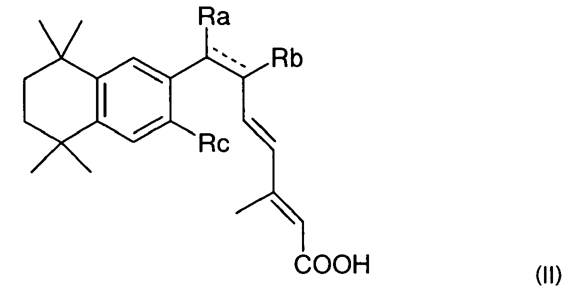 wherein the dotted line represents a bond (thus together with the solid line forming a double bond between the carbon atoms carrying Ra and Rb) or is absent (thus forming a single bond), and when the dotted bond is present, Ra is methyl and Rb is hydrogen, when the dotted bond is absent, Ra and Ra together are methylene thus forming, with the two carbon atoms carrying Ra and Rb, a preferably cis-substituted cyclopropyl ring; and Rc is C₁-C₄-alkoxy; the synthesis of such compounds is described e.g. in L.G. Hamman, J. Org. Chem. 65, 3233 (2000) and SS. Canan Koch et al., J. Med. Chem. 39, 3229 (1996);
or a compound of the formula III, 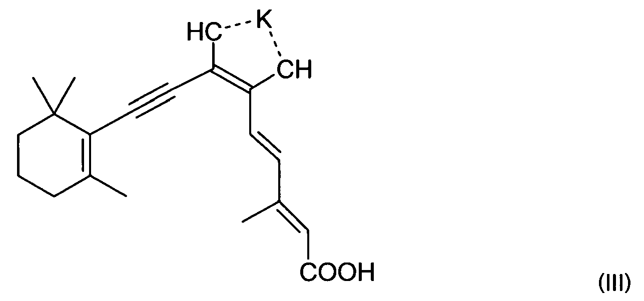
   wherein ----K---- is C₁-C₄-alkylene, especially -CH₂-CH₂-CH₂-, or =CH- CH= (thus together with the two carbon atoms binding ---K--- forming a benzene ring); and Rc is C₁-C₄-alkoxy; the synthesis of such compounds is described e.g. in EP 0 728 742 and US 5,986,131; or in each case a pharmaceutically acceptable salt thereof, or a pharmaceutically acceptable ester or a pharmaceutically acceptable amide, or in each of the two latter cases a pharmaceutically acceptable salt thereof.

Most preferred is the USE of a compound selected from the group consisting of Retinoid X Receptor (RXR) antagonists compound A, B, C, D, E, F and G listed in Table 1, or a pharmaceutically acceptable salt thereof, especially compound A or a pharmaceutically acceptable salt thereof.

**Table 1**

| Compound | Chemical Name |
|---|---|
| Compound A | (2E,4E,6Z)-7-[2-butoxy-3,5-bis(1,1-dimethylethyl)-phenyl]-3-methyl-2,4,6-octatrienoic acid |
| Compound B | (2E,4E,)-(1RS,2RS)-5-[2-(3,5-Di-tert-butyl-2-butoxyphenyl)-cyclopropyl]-3-methyl-penta-2,4-dienoic acid |
| Compound C | (2E,4E,)-(1RS,2RS)-5-[2-(3,5-Di-tert-butyl-2-ethoxyphenyl)-cyclopropyl]-3-methyl-penta-2,4-dienoic acid |
| Compound D | (2E,4E,6Z)-7-[3,5-Bis(1,1-dimethylethyl)-2-ethoxyphenyl]-3-methyl-2,4,6-octatrienoic acid ethyl ester |
| Compound E | (2E,4E)-3-Methyl-5-[2-(2,6,6-trimethyl-cyclohex-1-enylethynyl) -cyclohept-1-enyl]-penta-2,4-dienoic acid |
| Compound F | (2E,4E)-3-Methyl-5-[(1RS,2RS)-2-(5,5,8,8-tetramethyl-3- propoxy-5,6,7,8-tetrahydronaphthalen-2-yl)-cyclopropyl]-penta-2,4-dienoic acid |
| Compound G | (2E,4E,6Z)-3-Methyl-7-(5,5,8,8-tetramethyl-3-propoxy-5,6,7,8 -tetrahydro-naphthalen-2-yl)-octa-2,4,6-trienoic acid |

The expression "pharmaceutically acceptable salts" includes any salt chemically permissible in the art for retinoid antagonists that bear at least one salt-forming group, e.g. a basic group, such as amino, or an acidic group, such as carboxyl or sulfonyl, and that is applicable to warm-blooded animals, especially human beings (e.g. patients), for example in a pharmaceutically acceptable composition. Any conventional pharmaceutically acceptable salt of retinoid antagonists can be utilised. Among the conventional salts which can be made use of, there are the base salts included, for example, alkali metal salts such as the sodium or potassium salt, alkaline earth metal salts such as the calcium or magnesium salt, and ammonium or alkyl ammonium salts.

Where reference is made to a retinoid (e.g. RXR) antagonist within the present disclosure, this refers to the retinoid (e.g. RXR) acid antagonist, an ester or an amide thereof, each in free form and/or in the form of a pharmaceutically acceptable salt (= " a pharmaceutically acceptable amide, ester and/or salt thereof').

In accordance with this invention, it has been found that administration of a retinoid antagonist is efficacious in treating patients with inflammatory diseases of the skin and mucous membranes and of other tissues and organs.

The invention relates to the USE of a retinoid antagonist (this in a preferred embodiment of the invention relating to a RXR antagonist hereinbefore and hereinafter) where the disease of the skin and mucous membranes and of other tissues and organs to be treated is selected from the group of these diseases that can be treated with such antagonist.

Preferably, the diseases to be treated with such a retinoid antagonist are selected from one or more of the following diseases:
1. Inflammatory diseases of the skin, where any one or more of the following diseases is especially preferred:
   - psoriasis and its various types and forms,
   - an other keratinizing disorder,
   - Darier's disease,
   - lichen planus,
   - acne;
      more preferably:

   - allergic contact dermatitis (preferred) and/or eczema;
   - irritant contact dermatitis (preferred) and/or eczema;
      including the various clinical types and forms of endogenous, or more preferably exogenous (especially topic or after enteral or parenteral administration of e.g. medicaments or nutrients systemic) etiology and pathogenesis of the two mentioned disease groups of dermatitis and eczema.
2. Inflammatory diseases of mucous membranes, where any one or more of the following diseases are especially preferred:
   - a disease of the respiratory tract, especially laryngitis, (preferably non-allergic) bronchitis, or more preferably
   - an eye disease, especially:
      o blepharitis,
      o conjunctivitis, and
      o keratitis.
   - a nasal disease, especially rhinitis, preferably non-allergic rhinitis;
   - an ear disease, preferably otitis;
   - a disease or disorder of the digestive tract, especially pharyngitis, more especially stomatitis or proctitis;
   - a disease or disorder of the urogenital tract, especially urethritis, vulvitis, vaginitis or balanitis.
3. infectious diseases of skin and mucous membranes, induced by bacteria, fungi and/or viruses. In these diseases, retinoid antagonists are preferably used in combination with anti-infective agents, such as antibacterials, antibiotics, antifungals and/or anti-virals.
4. Especially inflammatory diseases of other tissues and organs than skin and mucous membranes, presenting signs, symptoms and lesions of inflammation, e.g. more preferably
4.1. T-helper cell type 1 (Th1)-mediated immune diseases, considered as autoimmune diseases or auto-reactive immune diseases; or diseases of mixed T-helper cell type (Th1)- and T-helper cell type 2 (Th2) or antibody-mediated immune diseases. The most important diseases of this category are:
   ■ Insulin-dependent diabetes mellitus ; more preferably:
   ■ multiple sclerosis, preferably in the acute phase; still more preferably:
   ■ rheumatoid arthritis;
   ■ systemic lupus erythematosus;
   ■ auto-immune thyroiditis, e.g. Hashimoto's disease;
   ■ Crohn's disease;
   ■ irritated bowel syndrome;
   ■ ulcerative colitis;
   ■ myasthenia gravis;
   ■ vasculitis,
   ■ diseases caused by immune complexes,
   ■ rejection of organ transplants and/or
   ■ graft versus host reaction.

### 4.2. Cancerous diseases with accompanying intra- or peritumoral inflammation.

Very often cancerous diseases are complicated with inflammatory reactions, which are sometimes very severe or even life threatening. These complications unfortunately cannot be overcome by conventional cancer therapy. Retinoid antagonists are therapeutically useful in treating intratumoral and peritumoral inflammation. This includes inflammation in primary tumors as well as in metastases. The anti-inflammatory therapy is especially useful as complementation (adjuvant therapy) to conventional cancer therapy before, during or after surgery, X-ray therapy, hormone therapy or chemotherapy.

### 4.3. (As a most preferred aspect of the invention) inflammatory diseases of the joints and/or bones, in particular rheumatoid arthritis (RA) and osteoarthritis (OA).

Conventional therapy of RA and OA is still dominated by non-steroidal anti-rheumatic drugs (NSARD) with Cox 1 or 2 specificity, corticosteroids immunosuppressive drugs such as methotrexate, and biologicals, such as TNF α antibody or TNF α receptor fusion protein. For various reasons such as lack of sufficient efficacy and incidence of adverse events, conventional therapy is not yet satisfactory. In addition, other inconveniences arise in the fact that the biologicals mentioned above cannot be administered orally but rather parenterally.

Especially important among all the diseases and/or symptoms where the USE according to the invention is made, there are non-allergic (that is especially e.g. infectious (infection-caused), autoimmune, mechanically induced) inflammations. Very especially important is or are also one or more inflammatory diseases not or not only based on the side effects of retinoid agonists, such as all-trans retinoic acid or 9-cis retinoic acid, that is preferably where the anti-inflammatory effect of a compound of the invention is other than a suppression of the toxic and inflammatory effect of a retinoid agonist, especially a retinoic acid agonist. The present invention for the first time shows that antiinflammatory effects of the (especially RXR) antagonists can be found in case of various different types of induction of inflammation. This shows that not merely the removal of side effects of agonists is achieveable by the administration of antagonists, but that a more general anti-inflammatory effect can be found with these compounds.

The finding that especially RXR antagonists (Retinoid X Receptor selective) are useful in treatment of inflammatory diseases of joints and bones is especially unexpected. Evidence is presented in this regard, especially in the examples, in a model system for rheumatoid arthritis and osteoarthritis. Human synovial fibroblast from fluid of joints of patients suffering from rheumatoid arthritis are co-cultured with human cartilage from persons with normal joints. RXR antagonists, such as compound A (see Table 1), inhibit the cartilage destruction in the test system employed in the examples, stimulated by the production of the cartilage destructive enzyme matrix metalloproteinase-1 (MMP-1).

The results obtained provide evidence for the inhibitory effect of RXR antagonists on cartilage degradation and destruction which is responsible for joint destruction. This beneficial effect on joint and bone diseases is particularly surprising as hitherto only retinoid agonists were (based on certain model systems such as Freund's adjuvant arthritis) regarded as useful in the treatment of rheumatoid arthritis. Therefore, the USE of retinoid antagonists, especially those mentioned as preferred, in the treatment of these diseases is a most preferred embodiment of the present invention.

In this respect the invention more particular, relates to USE against any one or more of the mentioned inflammatory diseases (especially those mentioned as preferred) or where inflammation is one component of such a disease.

The term "treatment" includes preventive (prophylactic) and/or especially therapeutic treatment. The compounds are being administered in an amount effective to treat that said disease or diseases, especially to a patient in need of such treatment.

For the treatment of the above mentioned diseases, the active compound, i.e. a retinoid antagonist, in particular a RXR antagonist, a pharmaceutically acceptable salt, or a pharmaceutically acceptable ester or amide thereof is administered either systemically or topically. Preferably, said active compound is administered as a composition containing said active compound and one or more pharmaceutically acceptable carrier or diluent compatible with said active compound. In preparing such composition, any conventional pharmaceutically acceptable carrier can be utilized. When the drug is administered orally, it is generally administered at regular intervals, conveniently at mealtimes or once daily. Based on information from toxicological studies, the retinoid antagonists are effective in doses which show no or only mild side effects when given orally or when given topically. Therefore, oral or topical administration of the active compound is generally preferred. For treating diseases of the skin, eye, ear, nose, the respiratory, digestive or urogenital tract, oral combined with topical administration may also be used advantageously.

In the treatment of the above-mentioned diseases, retinoid antagonists, when administered orally, do not or only slightly induce the adverse events belonging to the toxic syndrome of hypervitaminosis A, such as mucocutaneous, musculoskeletal, neurologic manifestations and elevation of transaminases, triglycerides and cholesterol. In addition, they are less teratogenic in contrast to the receptor agonistic retinoids clinically useful in the treatment of dermatological and oncological diseases, such as all-trans retinoic acid (tretinoin), 13-cis retinoic acid (isotretinoin), etretinate and acitretin.

In the treatment of inflammatory diseases of skin and mucous membranes, and of other tissues and organs, retinoid antagonists, pharmaceutically acceptable salts or pharmaceutically acceptable esters or amides thereof, can be used alone or in combination with other treatments, e.g. in combination with other pharmaceutically active substances such as topical or systemic corticosteroids, immunosuppressive drugs, antibacterial, antifungal or antiviral agents administered topically or systemically.

If used in combination with other substances, retinoid antagonists and said other substances can be administered separately, or incorporated in effective amounts into one pharmaceutical composition, or form a kit of parts the components of which may be administered at separate or overlapping times and/or at the same time.

The aforementioned retinoid antagonists, the salts and esters or amides thereof are especially useful especially in pharmaceutically acceptable oral or topical formulations. These pharmaceutical compositions comprise an active compound in association with a compatible pharmaceutically acceptable carrier material.

Any one or more conventional carrier materials suitable for oral administration can be used. Suitable carriers include water, gelatine, gum arabic, lactose, starch, magnesium stearate, talc, vegetable oils, polyalkylene-glycols, petroleum jelly and the like. Furthermore, the pharmaceutically active preparations may contain other pharmaceutically active agents. Additionally, additives such as flavouring agents, preservatives, complexing agents, pigments, dyes, stabilizers, tensides, emulsifying agents, wetting agents, solubilizers, buffers and the like may be added in accordance with accepted practices of pharmaceutical compounding.

The pharmaceutical preparations can be made up in any conventional form including inter alia: (a) a solid form for oral administration such as tablets, capsules (e.g. hard or soft gelatine capsules), pills, sachets, powders, granules, and the like; (b) preparations for topical administrations such as solutions, suspensions, ointment, creams, hydrogels, lipogels, micronized powders, sprays, aerosols and the like. The pharmaceutical preparations may be sterilized and/or may contain adjuvants such as preservatives, stabilizers, wetting agents, emulsifiers, salts for varying the osmotic pressure and/or buffers.

For topical administration to the skin or mucous membranes the aforementioned compounds are preferably prepared as ointments, tinctures, creams, gels, solution, lotions; nasal sprays; aerosols and dry powder for inhalation; suspensions, shampoos, hair soaps, perfumes and the like. In fact, any conventional composition can be utilized in this invention. Among the preferred methods of applying the composition containing the agents of this invention is in the form of an ointment, gel, cream, lotion; nasal spray; aerosol or dry powder for inhalation. The pharmaceutical preparation for topical administration to the skin can be prepared by mixing the aforementioned active ingredient with non-toxic, therapeutically inert, solid or liquid carriers customarily used in such preparation. These preparations preferably comprise 0.1 to 5.0 percent by weight, preferably 0.3 to 2.0 percent by weight, of the active compound, based on the total weight of the composition.

In preparing the topical preparations described above, additives such as preservatives, thickeners, perfumes and the like customary in the art of pharmaceutical compounding of topical preparation can be used. In addition, conventional antioxidants or mixtures of conventional antioxidants can be incorporated into the topical preparations containing the aforementioned active agent. Among the conventional antioxidants which can be utilized in these preparations are included N-methyl-α-tocopherolamine, tocopherols, butylated hydroxyanisole, butylated hydroxytoluene, ethoxyquin and the like. Cream-base pharmaceutical formulations containing the active agent, used in accordance with this invention, are composed of aqueous emulsions containing a fatty acid alcohol, semi-solid petroleum hydrocarbon, ethylene glycol and an emulsifying agent.

Ointment formulations containing the active agent in accordance with this invention, for example, comprise admixtures of a semi-solid petroleum hydrocarbon with a solvent dispersion of the active material. Cream compositions containing the active ingredient for use in this invention preferably comprise emulsions formed from a water phase of a humectant, a viscosity stabilizer and water, an oil phase of a fatty acid alcohol, a semi-solid petroleum hydrocarbon and an emulsifying agent and a phase containing the active agent dispersed in a aqueous stabilizer-buffer solution. Stabilizers may be added to the topical preparation. Any conventional stabilizer can be utilized in accordance with this invention. In the oil phase, fatty acid alcohol components function as a stabilizer. These fatty acid alcohol components function as a stabilizer. These fatty acid alcohol components are derived from the reduction of a long-chain saturated fatty acid containing at least 14 carbon atoms. Also, conventional perfumes and lotions generally utilized in topical preparation for the hair can be utilized in accordance with this invention. Furthermore, if desired, conventional emulsifying agents can be utilized in the topical preparations of this invention.

For topical treatment of diseases of mucous membranes of the respiratory tract, e.g. rhinitis and especially (preferably non-allergic) bronchitis, nasal sprays and inhalation aerosols are used. Formulations for such aerosols are described in Drugs and Pharmaceutical Sciences, Marcel Dekker, New York, 1996, Vol.72, pp. 547-574. Furthermore, the active compound can be delivered by dry powder inhalation. Examples for such formulations and devices are described in Pharmaceutical Technology, June 1997, pp. 117-125.

An example for a preferred oral dosage form comprises tablets, pills, sachets, or capsules of hard or soft gelatine, methylcellulose or of another suitable material easily dissolved in the digestive tract. Each tablet, pill, sachet or capsule can preferably contain from about 10 to about 500 mg, more preferably from about 20 to about 200 mg, of active ingredient. The oral dosages contemplated in accordance with the present invention will vary in accordance with the needs of the individual patient (e.g. the condition of the patient, the size, the age, possible interferences with other therapeutic measures and the like) as determined by the prescribing physician. Generally, however, a daily dosage of from 0.2 to 20 mg per kg of body weight, preferably 0.5 to 10 mg, and most preferably from about 1 mg to about 3 mg per kg of body weight of the patient is utilized. This dosage may be administered according to any dosage schedule determined by the physician in accordance with the requirements of the patient.

The dosage for treatment typically depends on the route of administration, the age, weight and disease condition of the individual. Suitable dosage forms are known in the art or can be easily obtained in a manner known per se. Formulations of lotions, gels, creams, sprays; aerosols and dry powder for inhalation, hard or soft gelatine capsules, tablets and sachets that are particularly suitable in the scope of the present invention can be easily adjusted in accordance with the above teaching in the art.

The pharmacological activity of the retinoid antagonists as disclosed above can be demonstrated in various test models as shown below, using the compounds: A, B, C, D, E, F and G, listed in Table 1.

Besides these mentioned dosage forms, also parenteral dosage forms (e.g. solutions or dispersions for injection and/or infusion) are envisable as useful in the invention, however, enterally administrable and/or topically administrable treatment and the corresponding dosage forms are preferred.

The following examples serve to illustrate the invention without limiting its scope:

### 1) Examples for inflammatory diseases of the skin

The experimental investigations for determining the anti-inflammatory effect of RXR antagonists refer to the following models for inflammatory diseases.

Examples for inflammatory diseases of the skin. Retinoid antagonists are tested for their anti-inflammatory properties.

### Examples 1 and 2: Acute and semichronic inflammation.

Inflammation is induced by topical (epicutaneous) application of retinoid receptor agonists e.g. retinoic acids all-trans retinoic acid (AtRA) or 9-cis retinoic acid (9-cis RA); or especially (this forming a case with a totally different etiology for the inflammation based on protein kinase C) by the topical application of the phorbolester 12-O-tetradecanoylphorbol-13-acetate (TPA).

### Methods

Nude mice of the C57BU6 strain are used. Inflammation is induced on mouse ears with either At RA, 9-cis RA or TPA by topical application. Inflammation is measured objectively by determination of the activity of myeloperoxidase (MPO) being directly correlated to the infiltration of polymorphonuclear white blood cells and by the determination of mRNA expression of c-jun, a protein implicated in the AP-1 transduction pathway according to known methods, see e.g. P.L. Stanley et al., Skin Pharmacol. 4, 262-271 (1991) (MPO assay), N. Basset-Sequin et al., J. Invest. Dermatol. 94, 418-422 (1990), F.J. Rauscher et al., Cell 52, 471-480 (1982), P- Sassone-Corn et al., Nature 326, 507-510 (1987) , and M. Pfahl, Endocr. Rev. 14, 651-658, 1993, which are incorporated by reference regarding the experimental method.

In the "acute inflammation" test, mice are treated topically (epicutaneously), orally or intraperitoneally, daily for 4 days. In the "semi chronic inflammation" test, mice are treated topically according to the schedules given below. For each experiment, a group of at least 4 mice of both sexes are used in the defined condition, regarding placebo, vehicle control, compound, topical formulation, oral formulation, dosage and concentration. The topical vehicle consists of ethanol/PEG 400/water (3:1:1).

### Results

### Example 1. Acute inflammation

The anti-inflammatory effect of topical RXR antagonists is tested by determination of myeloperoxidase activity in % of vehicle treated controls. For induction of inflammation 9-cis RA or TPA are applied topically to the skin, daily for 4 days. The RXR antagonist compound A (see Table 1) is administered topically one hour after the application of the inflammation inducing agent. The mice are sacrified 24 hours after the last treatment. The results are presented in Table 2:

**Table 2:**

| **Ears C57BL/6** | **MPO activity (% vehicle treated mice)** |
|---|---|
| 9-cis RA 0.05% (4d) | 647±142 |
| 9-cis RA 0.05% + compound A 0.05% (4d) | 236±92 |
| TPA 0.005% (4d) | 376±72 |
| TPA 0.005% + compound A 0.05% (4d) | 163±25 |
| TPA 0.005% + compound A 2.5% (4d) | 142±18 |

As can be seen from Table 2, topical administration of compound A significantly decreases the MPO activity induced by prior application of topical 9-cis RA or topical TPA.

### Example 2: Semi chronic inflammation.

The anti-inflammatory effect of topical RXR antagonists is tested by determination of myeloperoxidase activity in % of vehicle controls. The effect of the RXR antagonist compound A is also compared with the well known anti-inflammatory effect of the two corticosteroids, clobetasol dipropionate and betamethasone propionate. For induction of inflammation, AtRA and TPA are applied topically to the skin and the administration of the test compounds, the RXR antagonist compound A and the two corticosteroids are given in the following order, according to the schedule, described in: Skin Pharmacol 1991; 4 (4): 262-271, Stanley PL. et.al.: RA or TPA is administered on days 0, 2, 4, 7 and 9, compound A or corticosteroids are administered twice daily on day 7, day 8 and day 9 and once on day 10 in the morning. The mice are sacrificed on day 10 in the afternoon. The results are presented in Table 3.

**Table 3:**

| **Ears C57BL/6** | **MPO activity (% vehicle treated mice)** |
|---|---|
| At RA 0.05% (11d) | 1371±345 |
| At RA 0.05% + compound A 0.05% (11d) | 184±72 |
| TPA 0.005% (11 d) | 572±61 |
| TPA 0.005% + compound A 0.05% (11 d) | 345±81 |
| TPA 0.005% + Clobetasol dipropionate 0.05% (11d) | 239±43 |
| TPA 0.005% + Betamethasone propionate 0.05% (11 d) | 257±38 |

As can be seen from Table 3, topical administration of compound A significantly decreases the MPO activity induced by prior application of topical 9-cis RA or topical TPA. The two corticosteroids have a rather similar effect on the TPA-induced skin inflammation. This shows that the RXR antagonists do not only compensate the adverse effects of retinoic acid but are more generally applicable to really treat inflammations.

In the same experiment, the expression of c-Jun mRNA is determined by northern blot and expressed in percent of the vehicle controls. The results are presented in Table 4:

**Table 4:**

| **Ears C57BL/6** | **c-Jun mRNA expression (% of vehicle treated mice)** |
|---|---|
| Vehicle | 100 |
| TPA 0.005% | 163 |
| TPA 0.005% + Betamethasone 0.05% | 24 |
| TPA 0.005% + Clobetasol 0.05% | 43 |
| TPA 0.005% + compound A 0.05% | 88 |

As can be seen from Table 4, topical administration of compound A inhibits the c-Jun mRNA expression. The corticosteroids also decrease the expression of c-Jun mRNA. In the same concentration of 0.05% they have a stronger inhibitory effect than compound A. However, it has to be taken in consideration that compound A can be applied epicutaneously in much higher concentrations than the corticosteroids without inducing cutaneous adverse events.

TPA-induced inflammation is known to be transduced by AP-1 pathway. A partly common mechanism of action of RXR antagonists and corticosteroids may be possible, based on the repression of c-Jun expression and the correlated inhibition of myeloperoxidase.

### Example 3: Effect on normal, non-inflamed skin.

### Methods

The effect of the RXR antagonist compound A on normal skin of mice is investigated. The ears of C57BU6 mice are treated epicutaneously (topically) for 4 consecutive days with compound A in a concentration of 0.05 % and 2.5 % in acetone/ethanol (1:1, v/v). Comparison with vehicle control. Daily observation of skin reactions, in particular inflammation, erythema, desquamation, and edema follows. Myeloperoxidase (MPO) activity is determined. The activity of MPO is considered the most sensitive criterium for the assessment of inflammation of the skin (see above, Examples 1 and 1).

### Results

Compound A does not induce clinical signs or symptoms of a skin inflammation on normal skin of mice. At a concentration of 0.05 % of compound A, there is no significant chamge of MPO activity compared to the vehicle control. However, at a concentration of 2.5 % of compound A, MPO activity is significantly diminished to 57 % of that of the vehicle control. The conclusion may be drawn, that compound A in higher, but still well tolerated concentrations even decreases the basal activity of MPO in normal skin. This provides evidence to indicate a preventive effect of compound A towards inflammatory agents or in patients with inflammatory skin diseases in case of exposition to inflammation-inducing agents..

### Examples for inflammatory diseases of bones and joints.

### Example 4: Effect of RXR antagonists on degradation/destruction of human cartilage induced by synovial fibroblasts taken from patients with rheumatoid arthritis. Ex vivo, in vitro model system for rheumatoid arthritis (RA) and osteoarthritis (OA).

### Methods:

The effect of RXR antagonists on the activity of synovial fibroblasts, dependent on their state of activation, i.e. modified by a concomitant stimulation by the inflammatory cytokine Interleukin-1β (IL-1β), is determined. Furthermore, it is determined whether this is accompanied by a modulation in the accumulation of the mRNA encoding catabolic enzyme matrix metalloproteinase -1 (MMP-1), responsible for degradation of human cartilage and consequently joint destruction in man. Adherent synovial fluid cells taken from a patient with RA are used after 5 passages in an in vitro assay for cartilage destruction. The cells incubated in flasks coated with 0.1 % (0.1 g/100 ml) human cartilage powder are fixed using Matrigel® (BD Biosciences, Becton, Dickinson & Co., Boston, MS, USA) . The release of sulphated glycosaminoglycan (sGAG) into the culture medium is monitored by a commercial colorimetric test according to a method described by S. Björnsson, see Anal. Biochem. 256*,* 229-237 (1998) using an alcian blue dot plot analysis, and the accumulation of mRNA encoding MMP-1 is quantified by real time PCR (TaqMan® (Roche Diagnostics, Basle, Switzerland)).

The retinoid agonists all-trans retinoic acid and 9-cis retinoic acid, both physiological metabolites of vitamin A, as well as the RXR antagonist compound A, diluted first in ethanol, and then diluted with vehicle or medium to the are tested in a time course (0-35 days for the in vitro assay, 0-48 hours for MMP-1 mRNA, see tables 7, 8 and 10) and dose-dependent (10⁻⁷ to 10⁻⁹ M, see tables 5, 6 and 9). This is conducted in the presence or absence of IL-1β (100 pg/ml).

### Results

In the absence of IL-1β, the retinoid pan agonist 9-cis RA increases cartilage destruction in vitro in a dose-dependent manner (maximal between 10⁻⁷ M and 10⁻⁸ M), whereas the RXR antagonist compound A, in contrast, has no effect on the basal activity of synovial fibroblast (Table 5).

**Table 5:**

| **In vitro cartilage degradation. Dose dependency. Effect of 9-cis retinoic acid (9-cis RA) versus compound A (RXR antagonist) in absence of IL-1β. Release of sGAG in µg/ml/14 days.** | | |
|---|---|---|
| Dose/Concentration | sGAG in µg/ml | |
| | 9-cis RA | compound A |
| Vehicle control | 48 | 48 |
| 10⁻⁹ M | 64 | 46 |
| 10⁻⁸ M | 107 | 57 |
| 10⁻⁷ M | 189 | 39 |

However in the presence of IL-1β, quite surprisingly, the RXR antagonist compound A markedly inhibits the IL-1β dependent cartilage destruction, evidenced by a decrease in sGAG (Table 6).

**Table 6**

| **In vitro cartilage degradation. Dose dependency. Effect of 9-cis RA versus compound A (RXR antagonist) in presence of 100 pg/ml IL-1β. Release of sGAG in µg/ml/14 days.** | | |
|---|---|---|
| Dose/Concentration | sGAG in µg/ml | |
| | 9-cis RA | compound A |
| Vehicle control | 173 | 173 |
| 10⁻⁹ M | 204 | 144 |
| 10⁻⁸ M | 189 | 89 |
| 10⁻⁷ M | 221 | 41 |

The time course confirms that the retinoid agonist 9-cis RA markedly increases cartilage destruction in vitro, whereas with the retinoid antagonist compound A this is not the case. This effect is observed both in the presence and absence of IL-1β (Tables 7 and 8):

**Table 7:**

| **In vitro cartilage degradation. Time dependency. Effect of 9-cis RA versus compound A (RXR antagonist) in absence of IL-1β. Release of sGAG in µg/ml/14 days.** | | | |
|---|---|---|---|
| Days | Cumulative µg/ml sGAG/14 days | | |
| | Control | 9-cis RA 10⁻⁸ M | compound A 10⁻⁸ M |
| 7 | 23 | 44 | 44 |
| 14 | 48 | 107 | 57 |
| 21 | 84 | 206 | 57 |
| 28 | 112 | 253 | 39 |
| 35 | 117 | 292 | 34 |

**Table 8:**

| **In vitro cartilage degradation. Time dependency. Effect of 9-cis RA versus compound A (RXR antagonist) in presence of 100pg/ml IL-1β. Release of sGAG in µg/ml/14 days.** | | | |
|---|---|---|---|
| Days | Cumulative µg/ml sGAG/14 days | | |
| | Control | 9-cis RA 10⁻⁸ M | compound A 10⁻⁸ M |
| 7 | 86 | 68 | 67 |
| 14 | 173 | 189 | 89 |
| 21 | 212 | 330 | 89 |
| 28 | 249 | 407 | 173 |
| 35 | 271 | 441 | 56 |

Finally, the cartilage destruction in vitro correlates well with the accumulation of MMP-1 mRNA in synovial fibroblasts incubated for 12 hours. (Tables 9, 10):

**Table 9:**

| **Matrix metalloproteinase-1 (MMP-1) production. Dose dependency. Effect of 9-cis RA versus compound A (RXR antagonist) MMP-1 mRNA (real time PCR, fold increase of baseline value, after 24 hours) in relative units.** | | |
|---|---|---|
| Dose/Concentration | MMP-1 mRNA in relative units | |
| | 9-cis RA | compound A |
| Vehicle control | 1 | 1 |
| 10⁻⁹ M | 1.54 | 1.05 |
| 10⁻⁸ M | 3.39 | 1.12 |
| 10⁻⁷ M | 2.60 | 0.68 |

**Table 10:**

| **Matrix metalloproteinase-1 (MMP-1) production. Time dependency. Effect of 9-cis RA versus compound A (RXR antagonist) MMP-1 mRNA (real time PCR, fold increase of baseline value, after 0 to 24 hours) in relative units.** | | | |
|---|---|---|---|
| Hours | MMP-1 mRNA in relative units | | |
| | Control | 9-cis RA 10⁻⁸ M | compound A 10⁻⁸ M |
| 0 | 1 | 1 | 1 |
| 2 | | 0.98 | 1.13 |
| 6 | | 1.31 | 0.81 |
| 12 | 1.13 | 3.39 | 1.12 |
| 24 | | 3.79 | 0.87 |
| 48 | 1.09 | 1.47 | 0.83 |

### Conclusion

RXR antagonists inhibit cartilage destruction in a pharmacological model system for destruction of joints in rheumatoid arthritis and osteoarthritis.

### Examples of pharmaceutical formulations for treating inflammatory diseases by topical or oral administration of RXR antagonists.

The following formulations useful for USE in the present invention are prepared according to the tables presented and using standard procedures or the procedures specifically mentioned in Examples 5 to 8 for oral and in Examples 9 to 13 for topical administration, where "Active compound" stands for any one of compounds A, B, C, D, E, F and G mentioned in Table 1, preferably for compound A:

### Example 5: Fill mass for soft gelatin capsules and capsules filled with said fill mass:

A fill mass for soft gel capsules is prepared using the following components:

**Table 11:**

| **a) Fill mass for soft gelatin capsules** | |
|---|---|
| Active compound | 10-200 g |
| Oil* | 1-3 parts |
| Wax mixture** | 1-5 parts |
| Fill volume | 1-6 minims |
| | |
| * natural vegetable oils, e.g. soy oil, peanut oil, and artificial glycerides | |
| ** composition of natural and artificial waxes or partially hydrogenated fats | |

This fill mass is then used to produce soft gelatine capsules with the following content:

**Table 12:**

| **b) Soft gelatine capsules containing 20-100 mg active substance 20 mg soft gelatine capsule** | |
|---|---|
| Ingredients | **mg/capsule** |
| Active compound | 20.000 |
| dl-α-Tocopherol | 0.028 |
| Hydrogenated Castor Oil | 4.200 |
| Caprylic/Capric/Stearic Triglyceride (Synthetic Triglyceride) | 56.000 |
| Triglyceride, Medium Chain | 199.772 |
| Total | 280.000 mg |

### Example 6: Hard Gelatin capsules: Hard gelatine capsules are prepared as follows:

**Table 12:**

| **Hard gelatine capsules containing 20-100 mg active substance 20 mg hard gelatine capsule** | |
|---|---|
| **Ingredients** | **mg/capsule** |
| Active compound | 20.0 mg |
| Gelatine Bloom 30 | 70.0 mg |
| Maltodextrin MD 05 | 108.0 mg |
| dl-α-Tocopherol | 2.0 mg |
| Sodium ascorbate | 10.0 mg |
| Microcrystalline cellulose | 48.0 mg |
| Magnesium stearate | 2.0 mg |
| (weight capsule content) | 260.0 mg |
| | |
| **Procedure**: The active substance is wet milled in a solution of gelatine, maltodextrin, dl-α-Tocopherol and sodium ascorbate. The wet milled suspension is spray-dried. The spray-dried powder is mixed with microcrystalline cellulose and magnesium stearate. 260 mg each of this mixture are filled into hard gelatine capsules of suitable size and color. | |

### Example 7: Tablets: Tablets are prepared as follows:

**Table 13:**

| Tablets containing 20-50 mg active substance 20 mg tablet | |
|---|---|
| **Tablet kernel** | **mg/tablet** |
| Active compound | 20.0 mg |
| Anhydrous lactose | 130.5 mg |
| Microcrystalline Cellulose | 80.0 mg |
| dl-α-Tocopherol | 2.0 mg |
| Sodium ascorbate | 10.0 mg |
| Polyvinylpyrrolidone K30 | 5.0 mg |
| Magnesium stearate | 2.5 mg |
| (Kernel weight) | 250.0 mg |
| | |
| Film coat | |
| Hydroxypropyl methylcellulose | 3.5 mg |
| Polyethylenglycol 6000 | 0.8 mg |
| Talc | 1.3 mg |
| Irone oxide, yellow | 0.8 mg |
| Titanium dioxide | 0.8 mg |
| (weight of film) | 7.4 mg |
| | |
| **Procedure**: The compound is mixed with anhydrous lactose and microcrystalline cellulose. The mixture is granulated in water with a solution/dispersion of polyvinylpyrrolidone, dl-α-Tocopherol and sodium ascorbate. The granular material is mixed with magnesium stearate and afterwards pressed as kernels with 250 mg weight. The kernels are film coated with a solution/suspension of above-mentioned compositions. | |

### Example 8: Sachets: Sachets are prepared with the following ingredients:

**Table 14:**

| **Sachets containing 200-500 mg active substance 200 mg sachet** | |
|---|---|
| **Ingredients** | **mg/sachet** |
| Active compound | 200.0 mg |
| Lactose, fine powder | 990.0 mg |
| Microcrystalline Cellulose | 1250.0 mg |
| Sodium Carboxymethyl cellulose | 14.0 mg |
| dl-α-Tocopherol | 5.0 mg |
| Sodium ascorbate | 20.0 mg |
| Polyvinylpyrrolidone K30 | 10.0 mg |
| Magnesium stearate | 10.0 mg |

### Example 9: Lotion: A lotion is prepared with the following composition:

**Table 15:**

| **Lotion (solution)** | | |
|---|---|---|
| | | preferred |
| Active compound | 0.3-2.0 g | |
| Propylene Glycol | 5.00-20.00 g | 10.00 g |
| PEG- Glyceryl Cocoate* | 0.00-20.00 g | 10.00 g |
| dl-α-Tocopherol | 0.001-0.50 g | 0.02 g |
| Ascorbyl Palmitate | 0.01-0.20 g | 0.10 g |
| Propyl Gallate | 0.001-0.02 g | 0.002 g |
| Citric acid, anhydr.** | 0.00 -0.20 g | 0.01 g |
| Isopropanol*** | 40.00-90.00 g | 50.00 g |
| Water, dem. ad | 100.00 g | 100.00 g resp. ml |
| | | |
| * or other tensides | | |
| ** or other complexing agents e.g. EDTA | | |
| *** or other alcohols e.g. ethanol | | |

### Example 10: Gel: A gel is prepared with the following composition:

**Table 16:**

| Gel | | |
|---|---|---|
| | | preferred |
| Active compound | 0.3-2.0 g | |
| Propylene Glycol | 5.00-20.00 g | 10.00 g |
| PEG- Glyceryl Cocoate* | 0.00-20.00 g | 10.00 g |
| dl-α-Tocopherol | 0.001-0.50 g | 0.02 g |
| Ascorbyl Palmitate | 0.01-0.20 g | 0.10 g |
| Propyl Gallate | 0.001-0.02 g | 0.002 g |
| Citric acid, anhydr.** | 0.00 -0.20 g | 0.01 g |
| Isopropanol*** | 40.00-90.00 g | 50.00 g |
| HPMC**** | 0.50-5.00 g | 3.00 g |
| Preservative***** | q.s. | q.s. |
| Water, dem. ad | 100.00 g | 100.00 g resp. ml |
| * or other tensides | | |
| ** or other complexing agents e.g. EDTA | | |
| *** or other alcohols e.g. ethanol | | |
| **** Hydroxypropyl methylcellulose or other polymers e.g. neutralised Carbomer, Methyl cellulose, sodium carboxymethylcellulose | | |
| ***** Preservatives e.g. paraben esters (methyl, ethyl, propyl, butyl), sorbic acid and/or benzoic acid. | | |

### Example 11: Cream: A cream is manufactured with the following composition:

**Table 17:**

| Cream | | |
|---|---|---|
| | | preferred |
| Active compound | 0.3-2.0 g | |
| Glycerol | 0.00-10.00 g | 5.00 g |
| Na2 EDTA | 0.001-0.50 g | 0.03 g |
| Glycerides* | 5.00-20.00 g | 10.00 g |
| Cetyl Alcohol | 0.50-5.00 g | 1.00 g |
| Stearyl Alcohol | 0.50-5.00 g | 1.00 g |
| Glycerol mono Stearate | 1.00 -8.00 g | 4.00 g |
| Ceteareth** | 0.50-5.00 g | 2.00 g |
| dl-α-Tocopherol | 0.001-0.50 g | 0.02 g |
| Preservative*** | q.s. | q.s. |
| Water, dem. ad | 100.00 g | 100.00 g res. ml |
| | | |
| * e.g. caprylic/capric/triglyceride, caprylic/capric/linoleic triglycerides, natural glycerides, as well as e.g. propylene glycol, dicaprylate/dicaprate and waxes, such as stearyl, stearate, oleyl oleate, isopropyl myristate | | |
| ** Ceteareth 5-30, or other emulsifiers such as Polysorbate 20-80, sorbitane esters of fatty acids, fatty acid esters of PEG. | | |
| *** Preservatives, e.g. paraben esters (methyl, ethyl, propyl, butyl), sorbic acid and/or benzoic acid. | | |

### Example 12: Aerosol: An aerosol for inhalation with the following composition is filled into a metered dose inhaler

**Table 18:**

| Aerosol for inhalation, metered dose inhaler | |
|---|---|
| Active compound | 0.3-2.% |
| Sorbitantrioleate | 5 % |
| dl-α-Tocopherol | 0.4 % |
| Propellant (mixture of Trichlorofluoromethane and Dichlorodifluoromethane) | 94.1 % |

### Example 13: Drv powder for inhaler: A dry powder inhaler is filled with the following mixture:

**Table 19:**

| Dry powder inhaler | |
|---|---|
| Active compound (jet-milled, spray-dried) | 0.3-2.0 mg |
| Lactose monohydrate | 25 mg |

### Examples for the effect of compound A (RXR antagonist) administered topically to the skin of a healthy human volunteer. Clinical Pilot Trial. Epicutaneous application.

### Example 14: Effect of topical compound A on healthy, non-inflamed human skin.

Topical application of compounds to the skin is frequently handicapped by inflammation of human skin. Compound A (RXR antagonist) is therefore tested for its inflammation potential on human skin.

### Methods

In a clinical pilot trial on one healthy volunteer compound A is applied epicutaneously in a concentration of 1 % for its inflammation potential compared to that of the strong inflammation inducing agent 9-cis RA in concentrations of 0.1 %, 0.3% and 1%.

The compounds are solved or suspended in ethanol/propylene glycol (1:1). They are applied epicutaneously twice daily, 7 days a week, for two concecutive weeks. The site of application is an area of 3x3 = 9cm² on the abdominal skin. The volume is 0.1 ml per application.

The following signs and symptoms of skin inflammation are recorded: Erythema, desquamation (scaling), pruritus, burning, pain, exsudation, edema, ulceration. They are classified on a scale of:
0 = no signs or symptoms of skin inflammation
1 = slight erythema, desquamation and pruritus
2 = moderate erythema, desquamation and pruritus
3 = marked erythema, desquamation, pruritus/burning
4 = severe erythema, desquamation, pruritus, burning or even pain, edema, exsudation, ulceration

Treatment period lasts from day 1 to 14, the post-treatment observation period from day 15 to day 28

### Results

9-cis RA is tested in three concentrations: 0.1 %, 0.3% and 1.0%. During the first 9 days after start of treatment no signs or symptoms of skin inflammation are observed. Around the tenth and eleventh day, the symptoms become manifest in the form of slight erythema, desquamation and pruritus. These symptoms increase during days 12, 13 and 14, depending on the concentration, to moderate inflammation with 0.1 and 0.3% and to marked inflammation with 1.0%. Three days after discontinuation of treatment i.e. on day 17, the inflammation still persists and is concentration dependent: Marked inflammation (3) with 1%, moderate (2) with 0.3% and slight (1) with 0.1 %. From day 18 on, inflammation decreases to 0, depending on the concentration, between days 18 and 22, i.e. 4 to 8 days after discontinuation of treatment.

Compound A is tested at a concentration of 1 %. In contrast to 9-cis RA which induces a marked skin inflammation at 1% concentration, compound A is well tolerated with no skin inflammation at 1 % concentration. Compound A does not induce any objective or subjective symptoms, neither during the treatment period, nor during the post-treatment observation period.

### Conclusion:

Compound A applied epicutaneously to human skin does not induce signs or symptoms of inflammation of the skin in a 28 days clinical pilot study.

### Example 15: Therapeutic effect of topical compound A on human skin inflammation induced by topical 9-cis retinoic acid (9-cis RA).

Compound A (RXR antagonist) applied epicutanously is tested on its anti-inflammatory effect on human skin, in which inflammation has been induced by topical 9-cis RA.

### Methods

In the following pilot clinical trial on one healthy volunteer (WB) the compounds are applied epicutaneously. 9-cis RA being known for its skin inflammation potential is used in concentrations of 0.1 %, 0.3% and 1%. Compound A (RXR antagonist) concentration is 1%. The compounds are solved in ethanol/propylene glycol (1:1). 9-cis RA is applied twice daily, 7 days a week, for two consecutive weeks. The site of application is an area of 3x3 = 9 cm² on the abdominal skin. The volume is 0.1 ml per administration.

The treatment with compound A, administered twice daily in a concentration of 1 %, is started on day 15 when treatment with 9-cis RA is discontinued. This treatment lasts from day 15 to day 22.. For a correct evaluation comparative areas with inflammation induced by 9-cis RA are treated with the vehicle, ethanol/propylene glycol, from day 15 to 22. The post-treatment period lasts until day 28.

The signs and symptoms of skin inflammation are recorded on a 0-4 scale, as described in example 14.

For evaluation of the anti-inflammatory effect of compound A, the sum of the daily inflammation scores from day 15 to day 28 is determined, as well as the time to complete disappearance of skin inflammation from day 15 on.

### Results (see Table 20)

### Anti-Inflammatory Effect of RXR Antagonist Compound A on 9-cis Retinoic Acid (9-cis RA)-Induced Skin Inflammation - Clinical Pilot Trial

Induction of skin inflammation: Topical application of 9-cis RA 0.1 %, 0.3 % and 1 %
- Therapy:: Topical application of compound A 1 %
- Comparison:: 9-cis RA 0.1 %, 0.3 % and 1 % day 1-14, followed by vehicle, day 15-22 9-cis RA 0.1%, 0.3 % and 1 % day 1-14, followed by compound A 1 %, day 15-22
- Skin inflammation:: scale 0-4, daily determination from baseline to day 28

**Table 20:**

| Retinoids | Sum of daily inflammation scores from day 15 to disappearance of inflammation | Time in days from day 15 to disappearance of inflammation |
|---|---|---|
| 9-cis RA 0.1% | 6.5 | 8 |
| 9-cis RA 0.1% +compound A 1% | 2.5 | 3 |
| | | |
| 9-cis RA 0.3 % | 14 | 13 |
| 9-cis RA 0.3% +compound A 1 % | 5.5 | 6 |
| | | |
| 9-cis RA 1 % | 14.5 | 9 |
| 9-cis RA 1 % +compound A 1 % | 11 | 8 |

9-cis RA given topically exerts a significant skin inflammatory effect. The induction of inflammation is dependent on the concentration of 9-cis RA. A solution of 1 % 9-cis RA provokes a much higher skin irritation than that of 0.1 %.

After the treatment with 9-cis RA within the first 2 weeks, inflammation tends to decrease and to disappear between day 15 and day 23.

The time to disappearance of inflammation is markedly shortened when compound A in a concentration of 1 % is applied between day 15 and day 22, compared with the vehicle control. In the case of 0.1 % 9-cis RA induction of skin inflammation the time to disappearance of inflammation is 3 days, when compound A is administered in a 1% concentration, compared to 8 days in the case of vehicle application.

This anti-inflammatory effect is also evidenced by determination of the sum of daily inflammation scores from day 15 to day 28. By the treatment with 1 % of compound A, the sum of daily inflammation scores in the 0.1 % 9-cis RA study is reduced to 2.5, compared to 6.5 by treatment with the vehicle control.

The study with 0.3% 9-cis RA gives rather similar effects as the study with 0.1 % 9-cis RA. When inflammation is induced by the high concentration of 1 % 9-cis RA the anti-inflammatory effect is less significant, with a reduction of the sum of daily inflammation scores from 14.5 to 11.

### Example 16: Anti-Inflammatory Effect of RXR Antagonist Compound A on 9-cis RA Induced Skin Inflammation - Comparison of Preventive and Therapeutic Effect of Compound A

This human volunteer study represents an additional study with regard to example 15. Example 15 deals with a study wherein the therapeutic anti-inflammatory effect of compound A is demonstrated by the administration of topical compound A after the skin inflammation has been induced before, by topical administration of 9-cis RA. The present study is carried out for comparing the preventive and the therapeutic effect of the RXR antagonist compound A on skin inflammation induced by topical 9-cis RA.

### Methods

In this pilot clinical trial on one healthy volunteer (WB), the substance 9-cis RA and compound A are administered epicutaneously. The inflammation-inducing agent 9-cis RA is used in a concentration of 0.3%. The RXR antagonist compound A is applied in a concentration of 1%. The compounds are solved in ethanol/propylene glycol (1:1) and administered twice daily. The site of application is the abdominal skin, with various areas of 3 x 3= 9cm². Volume per application is 0.1ml.

The following three clinical settings at different sites are chosen:

### 1. Induction of skin inflammation by 9-cis RA

9-cis RA is administered as a 0.3% solution twice daily from day 1 to day 14. The vehicle is administered from day 15 to disappearances of skin inflammation.

### 2. Prevention of 9-cis RA-induced skin inflammation by RXR antagonist Compound A

9-cis RA solution of 0.3% is administered twice daily from day 1 to day 14, each time followed subsequently by application of compound A as a 1% solution

### 3. Therapy of 9-cis RA-induced skin inflammation by RXR antagonist compound A

9-cis RA solution of 0.3% is administered twice daily from day 1 to day 14. Compound A as a 1 % solution is administered from day 15 until disappearance of skin inflammation.

The signs and symptoms of skin inflammation are recorded on a 0-4 scale, as described in example 14. The evaluation of the anti-inflammatory effect is based on the daily determination of the inflammation score (scale 0-4).

The following parameters serve as criteria for evaluation of the effect of 9-cis RA, the preventive effect of compound A and the therapeutic effect of compound A.
1. Sum of the daily inflammation scores from day 1 to day 28. Total inflammation score.
2. Sum of the daily inflammation scores from day 15 to disappearance of skin inflammation.
3. Time in days from day 15 to disappearance of skin inflammation.

### Results (Table 21):

**Table 21:**

| Retinoids | Sum of daily inflammation scores, day 1 to day 28. Total inflammation score | Sum of daily inflammation scores, from day 15 to disappearance of inflammation | Time in days from day 15 to disappearance of inflammation |
|---|---|---|---|
| 9-cis RA 0.3 %, day 1 to day 14 | 18 | 14 | 13 |
| | | | |
| Prevention 9-cis RA 0.3 %, day 1 to day 14 compound A 1 %, day 1 to day 14 | 11 | 5 | 6 |
| | | | |
| Therapy 9-cis RA 0,3 % , day 1 to day 14 compound A 1 %, day 15 to disappearance of inflammation | 9 | 5 | 6 |

9-cis RA has a marked inflammatory effect on human skin. The total inflammation score is 18. The sum of daily inflammation scores from day 15 to complete disappearance is 14. 13 days are needed from day 15 on to complete disappearance of skin inflammation.

### Prevention of skin inflammation by the RXR antagonist compound A

Compound A has a marked effect. All parameters for evaluation are influenced. In this prevention trial the total inflammation scores decrease from 18 to 11, the sum of daily inflammation scores from day 15 to disappearance of skin inflammation decreases from 14 to 5 and the time from day 15 to disappearance of skin inflammation from day 15 to disappearance of skin inflammation decreases from 13 days to 6 days.

### Therapy of skin inflammation by the RXR antagonist compound A

Compound A has a marked-inflammatory effect in this therapeutic clinical trial. All parameters are reduced by 50% or more in comparison to the values of the inflammation-inducing agent 9-cis RA. Total inflammation score decreases from 18 to 9, the sum of daily inflammation scores from day 15 until disappearance of skin inflammation is reduced from 14 to 5 and the time from day 15 to disappearance of skin inflammation decreases from 13 to 6 days.

### Conclusion

The results of examples 15 and 16 represent a clinical proof of concept for the efficacy of the RXR antagonist compound A as an anti-inflammatory agent in prevention and therapy of inflammatory diseases, in particular inflammatory diseases of the skin.

### Example 17: Effect of compound A administered to the skin of healthy volunteers where skin inflammation is induced by topical application of Candidin (extract of Candida albicans) or UV-B irradiation

Clinical phase I trial, approved by the Ethical Committee of the University Hospital of Geneva and by Swiss Drug Agency Swiss Medic.

In this clinical phase I trial, the anti-inflammatory effect of compound A and that of conventional therapy with topical corticosteroids or immunomodulatory macrolides are compared. In contrast to the examples 15 and 16, skin inflammation in example 17 is not induced by a retinoid agonist such as 9-cis retinoic acid. The anti-inflammatory effect of the retinoid antagonist compound A (RXR antagonist) can therefore not be considered merely as suppression of the toxic and inflammatory effect of a retinoid agonist.

Further evidence of the large difference between the inflammatory effect of a retinoid agonist and that of the other inflammation-inducing agents is the fact that their spectrum of toxic side effects is highly different. Retinoid agonists, when administered systemically, induce the typical hypervitaminosis A syndrome, manifesting itself in headache, flushes, cheilitis, conjunctivitis, various other mucocutaneous manifestations, musculoskeletal symptoms and laboratory abnormalities, such as elevation of transaminases, triglyerides and cholesterol. The skin inflammation-inducing agents used in example 17 do not induce this spectrum of toxic side effects. Thus example 17 is a clinical proof for the unexpected and non-obvious inventive anti-inflammatory usefulness of the group of RXR antagonistic compounds.

### Methods:

Four healthy volunteers participate. Inclusion criteria are: Age above 18 years, male or female. They are informed, with written letter of consent. Exclusion criteria are:

Preexistant dermatological diseases, known allergy to test agents.

Inflammation inducing agents: Candidin is administered intradermally (see also D. Poffet, Comparaison entre le pouvoir vaso-constricteur d'un corticoide topique et l'inhibition de la dermite à la candidine après intradermoréaction (IDR). Thèse, Université de Genève, 1984). UV-B rays are administered by a UV-B lamp.

Inflammation is measured quantitatively. The area of the inflamed skin is measured in cm². The thickness of the skin is monitored by Ultrasound at 20 MHz. Erythema is measured by colorimetric determination employing a Minolta CR 20.

The following anti-inflammatory agents are used:
- Compound A (RXR antagonist): Lotion, concentration of 1 % active ingredient dissolved in ethanol/PEG 400/water (3:1:1).
- Corticosteroids: Diprosone® (Essex Pharma; active principle: betamethasone dipropionate) cream; Dermovate® (GlaxoSmithKline; active principle: clobetasol propionate) cream.
- Macrolides: Protopic® (Fujisawa; active principle: tacrolimus) cream; Elidel® (Novartis; active principle: pimecrolimus) cream.

### Plan of the study:

Area of administration: Six separate small skin areas on each forearm of every volunteer.

Day 1: Determination of skin thickness. Administration of the inflammatory agents (only on day 1). Administration of compound A, corticosteroids or macrolides on the area where inflammatory agents had been placed immediately before. One area is treated by vehicle control or not treated at all.
Day 2: Determination of skin thickness, erythema and area of inflamed skin. Application of anti-inflammatory test substances.
Day 3: Determination of skin thickness, erythema and area of inflamed skin. Application of anti-inflammatory test substances.
Day 4: Determination of skin thickness, erythema and area of inflamed skin.

All determinations are recorded on a scale from 0 to 4:
- 0 =: no reduction
- 1 =: slight reduction (10 to 20 %)
- 2 =: moderate reduction (21 to 40 %)
- 3 =: marked reduction (41 to 70 %)
- 4 =: very marked reduction (71 to 100 %)

Compared to vehicle control.

### Results:

In the 4 volunteers a marked to very marked reduction of the inflammatory reaction is found when treated by corticosteroids or immunomodulatory macrolides. In the volunteers treated with topical compound A, the anti-inflammatory effect is even superior to that of corticosteroids or macrolides. The reduction of the inflammatory reaction is very marked in all volunteers treated with topical compound A. A further advantage of compound A is the fact that in earlier pilot trials compound A is devoid of any side effects (see also example 14) on the skin, even when applied topically not only for 4 days but even for 14 to 20 days. This is not always the case with corticosteroids and immuno-modulatory macrolides.

Conclusion: Compound A administered topically has been demonstrated to be a clinically efficacious anti-inflammatory agent, superior to conventional therapy with topical corticosteroids or immunomodulatory macrolides. A further advantage of topical compound A is its lack of inducing adverse side effects on the skin. The results in the pilot trials of example 17 on humans correspond to the results achieved in the animal experiment reported in examples 1 and 2 given above.

## Claims

1. The use of a retinoid antagonist, a pharmaceutically acceptable ester or amide thereof or pharmaceutically acceptable salt of any of these for the manufacture of a pharmaceutical preparation for the treatment of a disease of the skin and/or a mucous membrane or other diseases of the skin and/or mucous membranes, wherever inflammation is one component of the disease manifestations, which comprises administering to a subject having an inflammatory disease of the skin or mucous membranes or another disease of the skin and mucous membranes, wherein inflammation is one component of the disease manifestations.

2. The use according to claim 1 where the retinoid antagonist is a retinoid RXR antagonist compound selected from the group consisting of a compound of the formula I, 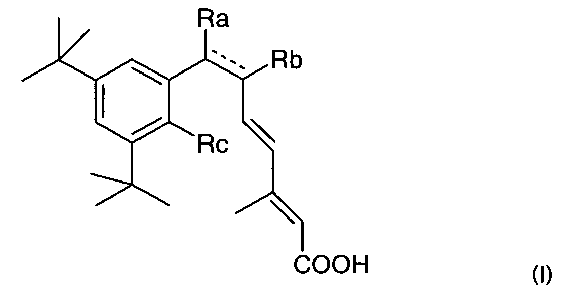 wherein the dotted line represents a bond thus together with the solid line forming a double bond between the carbon atoms carrying Ra and Rb or is absent thus forming a single bond, and when the dotted bond is present, Ra is methyl and Rb is hydrogen, when the dotted bond is absent, Ra and Rb together are methylene thus forming, with the two carbon atoms carrying Ra and Rb, a preferably cis-substituted cyclopropyl ring; and Rc is C₁₋C₄-alkoxy;
a compound of the formula II, 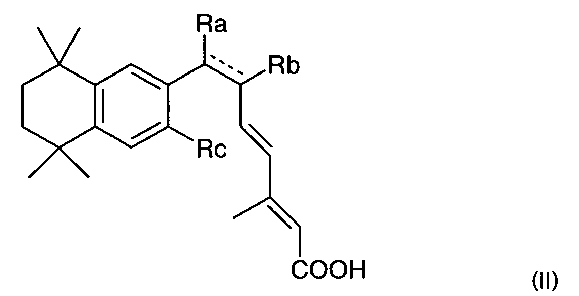 wherein the dotted line represents a bond thus together with the solid line forming a double bond between the carbon atoms carrying Ra and Rb or is absent thus forming a single bond, and when the dotted bond is present, Ra is methyl and Rb is hydrogen, when the dotted bond is absent, Ra and Rb together are methylene thus forming, with the two carbon atoms carrying Ra and Rb, a preferably cis-substituted cyclopropyl ring; and Rc is C₁₋C₄-alkoxy;
and a compound of the formula III, 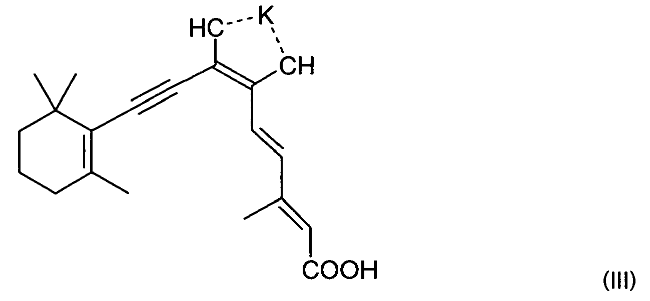 wherein ----K---- is C₁-C₄-alkylene, especially -CH₂-CH₂-CH₂-, or is =CH-CH= thus together with the two carbon atoms binding ---K--- forming a benzene ring; and Rc is C₁-C₄-alkoxy;
or in each case a pharmaceutically acceptable amide, ester and/or salt thereof.

3. The use according to any one of claims 1 or 2 wherein the retinoid antagonist is an RXR antagonist selected from the group consisting of
(2E,4E,)-(1RS,2RS)-5-[2-(3,5-di-tert-butyl-2-butoxy-phenyl)-cyclopropyl]-3-methyl-penta-2,4-dienoic acid,
(2E,4E,)-(1RS,2RS)-5-[2-(3,5-di-tert-butyl-2-ethoxy-phenyl)-cyclopropyl]-3-methyl-penta-2,4-dienoic acid,
(2E,4E,6Z)-7-[3,5-bis(1,1-dimethylethyl)-2-ethoxyphenyl]-3-methyl-2,4,6-octatrienoic acid ethyl ester,
(2E,4E)-3-methyl-5-[2-(2,6,6-trimethyl-cyclohex-1-enylethynyl) -cyclohept-1-enyl]-penta-2,4-dienoic acid,
(2E,4E)-3-methyl-5-[(1RS,2RS)-2-(5,5,8,8-tetramethyl-3- propoxy-5,6,7,8-tetrahydronaphthalen-2-yl)-cyclopropyl]-penta-2,4-dienoic acid,
(2E,4E,6Z)-3-methyl-7-(5,5,8,8-tetramethyl-3-propoxy-5,6,7,8 -tetrahydro-naphthalen-2-yl)-octa-2,4,6-trienoic acid
and especially (2E,4E,6Z)-7-[2-butoxy-3,5-bis(1,1-dimethylethyl)phenyl]-3-methyl-2,4,6-octatrienoic acid;
or in each case a pharmaceutically acceptable amide, ester and/or salt thereof.

4. The use according to any one of claims 1 to 3 wherein the disease is T-helper cell type 1 or mixed T-helper cell type 1/T-helper cell type 2 mediated.

5. The use according to any one of claims 1 to 4 where the inflammation symptoms associated with the disease are to be treated.

6. The use according to any one of claims 1 to 5 wherein the disease to be treated is a disease of the skin which is allergic eczema and/or preferably allergic dermatitis, especially contact dermatitis.

7. The use according to any one of claims 1 to 5 wherein the disease to be treated is a disease of the skin which is an irritant contact eczema and/or preferably an irritant contact dermatitis.

8. The use according to any one of claims 1 to 5 wherein the disease to be treated is a disease of the skin which is eczema or dermatitis of exogenous etiology, respectively.

9. The use according to any one of claims 1 to 5 wherein the disease to be treated is a disease of the skin which is one of the various types and forms of psoriasis, another keratinizing disorder, or Darier's disease or lichen planus.

10. The use according to any one of claims 1 to 5 wherein the disease to be treated is a disease of the skin which is acne, preferably acne with an inflammatory component.

11. The use according to any one of claims 1 to 5 or any one of claims 6 to 10 wherein the disease to be treated is a disease of the skin which is an infectious disease.

12. The use according to any one of claims 1 to 5 wherein the disease to be treated is a disease of a mucous membrane which is a disease of the respiratory tract, especially laryngitis, and/or bronchitis, especially non-allergic bronchitis.

13. The use according to any one of claims 1 to 5 wherein the disease to be treated is a disease of the mucous membrane which is an eye disease, preferably blepharitis, conjunctivitis and/or keratitis.

14. The use according to any one of claims 1 to 5 wherein the disease to be treated is a disease of the mucous membrane which is a nasal disease, preferably rhinitis, especially non-allergic rhinitis, or preferably an ear disease, especially otitis.

15. The use according to any one of claims 1 to 5 wherein the disease to be treated is a disease of the mucous membrane which is a disease or disorder of the digestive tract, preferably pharyngitis, more preferably stomatitis or proctitis.

16. The use according to any one of claims 1 to 5 wherein the disease to be treated is a disease of the mucous membrane which is a disease or disorder of the urogenital tract, especially urethritis, vulvitis, vaginitis and/or balanitis.

17. The use according to any one of claims 1 to 5 and 12 to 16 wherein the disease to be treated is a disease induced by bacteria, fungi and/or viruses.

18. The use according to any one of claims 1 to 5 where the disease to be treated is a disease or disorder of the skin and/or mucous membrane and the pharmaceutical preparation is a combination in the form of a fixed combination or a kit of parts for the simultaneous, separate or sequential use comprising a retinoid RXR antagonist and one or more other agents selected from the group consisting of anti-inflammatory agents and anti-infective agents, especially antibacterial, anti-fungal and or anti-viral agents and is especially for oral or topical administration.

19. The use according to any one of claims 1 to 5 wherein the disease to be treated is an inflammatory disease of a tissue and/or organ apart from skin and mucous membranes or a disease of a tissue and/or organ apart from skin and mucous membranes, wherein inflammation is one component of the disease manifestations.

20. A use according to claim 19 wherein the disease is a cell-mediated immune disease and/or a T-helper cell type 1 mediated immune disease or an autoimmune disease.

21. The use according to any one of claims 19 and 20 where the disease to be treated is multiple sclerosis, especially in the acute phase.

22. The use according to any one of claims 19 and 20 where the disease to be treated is insulin-dependent diabetes mellitus.

23. The use according to any one of claims 19 and 20 where the disease to be treated is rheumatoid arthritis, systemic lupus erythematosus, an auto-immune disease, especially auto-immune thyroiditis, Crohn's disease, irritable bowl syndrome, ulcerative colitis, myasthenia gravis, vasculitis, a disease caused by immune complexes, acute and/or chronic rejection of an organ transplant and/or a graft versus host reaction.

24. A use according to any one of claims 19 or 20, wherein the inflammatory disease is a peritumoral or intratumoral inflammation occuring in a cancerous disease, such as a carcinoma or a sarcoma of any kind of tissues and organs of the body including primary tumors and/or metastases.

25. A use according to any one of claims 19 and 20 wherein the inflammatory disease is an inflammation of joints and/or bones, especially rheumatoid arthritis or osteoarthritis, especially an inflammatory disease leading to cartilage destruction, joint destruction and/or bone destruction.

26. The use according to any one of claims 2 to 18 or especially 1 or 19 to 25 wherein the pharmaceutical preparation is for oral administration, especially at a daily dosage of from about 0.2 mg to about 20 mg, more preferably 1.0 to 5 mg of the compound per kg of body weight of the subject.

27. The use according to claim 26 wherein the pharmaceutical preparation is prepared in the form of a tablet a capsule, a pill or a sachet comprising from 10 to 500 mg, preferably from 20 to 200 mg of the retinoid antagonist.

28. The use according to any one of claims 2 to 18 or especially 1 or 19 to 25 wherein the pharmaceutical preparation is for topical administration, especially an ointment, a cream, a lotion, a gel or a spray comprising from 0.1 to 5.0, preferably from 0.3 to 2.0, percent by weight of the retinoid antagonist.

29. The use according to claim 28 wherein the pharmaceutical preparation comprises one for inhalation, preferably is a nasal aerosol, an aerosol for inhalation or a dry powder for inhalation, each comprising from 0.1 to 5, more preferably 0.3 to 2.0, percent by weight of the retinoid antagonist.

30. The use according to any one of claims 19 to 29 wherein the pharmaceutical preparation is a combination in the form of a fixed combination or a kit of parts for the simultaneous, separate or sequential use comprising a retinoid RXR antagonist and one or more other agents selected from the group consisting of anti-inflammatory agents and anti-infective agents, and is especially for oral or topical administration.

31. The use of a retinoid antagonist, a pharmaceutically acceptable ester, a pharmaceutically acceptable amide and/or a pharmaceutically acceptable salt thereof, especially one as shown in any of claims 2 or 3, in the treatment of a disease or disorder mentioned in any one of claims 1 or 4 to 25, preferably in the doses given in claim 26, preferably in the form of a pharmaceutical preparation mentioned in any one of claims 18 or 26 to 30, or a retinoid antagonist, a pharmaceutically acceptable ester, a pharmaceutically acceptable amide and/or a pharmaceutically acceptable salt thereof, especially one as shown in any of claims 2 or 3, for use in the treatment of a disease or disorder mentioned in any one of claims 1 or 4 to 25, preferably in the doses given in claim 26, preferably in the form of a pharmaceutical preparation mentioned in any one of claims 18 or 26 to 30.

32. A method of treatment for a disease or disorder mentioned in any one of claims 1 or 4 to 25, comprising administering to a mammal, especially a human, in need of such treatment an amount of a retinoid antagonist, a pharmaceutically acceptable ester, a pharmaceutically acceptable amide and/or a pharmaceutically acceptable salt thereof, especially as shown in any one of claims 2 or 3, that is efficient in the treatment of said disease, preferably in a dose according to claim 26.

33. A pharmaceutical preparation for the treatment of a disease or disorder mentioned in any one of claims 1 or 4 to 25, comprising a retinoid antagonist, a pharmaceutically acceptable ester, a pharmaceutically acceptable amide and/or a pharmaceutically acceptable salt thereof, especially as shown in any one of claims 2 or 3, and a pharmaceutically acceptable carrier material.
